# EUROPEAN PATENT APPLICATION

(11) **EP 4 471 120 A1**
(43) Date of publication of application: **04.12.2024**
(21) Application number: 23176761.7
(22) Date of filing: 01.06.2023
(51) Int. Cl.: C12M 1/107, C02F 11/04, C02F 11/10, C02F 11/13, C12M 1/36, C12M 1/00, C12P 5/02

(54) **BIOGAS PRODUCTION SYSTEM AND OPERATION CONTROL METHOD**

(71) Applicant: Kanadevia Inova AG, 8005 Zürich (CH)
(72) Inventor: SCHNEIDER, Adrian, 8005 Zürich (CH); HACK, Gamuret, 8057 Zürich (CH); MOIOLI, Emanuele, 5200 Brugg (CH); HÄHNLE, Hartmut, 5408 Ennetbaden (CH)
(74) Representative: Schaad, Balass, Menzl & Partner AG

(57) **Abstract**

The present application relates inter alia to a biogas production system including
an anaerobic digester 10 configured for the production of biogas 12 and a digestate 14 from an organic feedstock 16;
a separator 20, 21 configured for separating at least part of the digestate 14 from the digester 10 into a liquid digestate fraction 24 and a solid digestate fraction 22;
a thermal treatment unit 30configured for thermally treating at least part of the solid digestate fraction 22;
a controller 38 configured for controlling an operational parameter of the separator 20, 21 in response to at least one measured variable that is indicative of the energy demand and/or the energy production of the system; and
a sensor device 39 configured for measuring the at least one variable.

## Description

The present invention relates to a biogas production system according to Claim 1 and to a method for operating such a biogas production system according to Claim 11.

Biogas as a clean and CO₂-neutral energy carrier can make an important contribution to increase renewable energy's share in energy supply. One key driving force for biogas production is the reduction of greenhouse gas (GHG) emissions by the substitution of fossil fuels. Biogas is commonly produced from anaerobic digestion of various digestible organic substrates and wastes or from landfills. The biogas can be used for different energy services, such as heat, combined heat and power (CHP), vehicle fuel, and natural gas grid after upgrading.

Apart from biogas anaerobic digestion also produces a digestate, which is the residual material left after the digestion process and is composed of liquid and solid portions. These liquid and solid portions or fractions are often separated and handled independently, as each have value that can be realized with varying degrees of post-processing. In particular, with appropriate treatment, both the solid and liquid portions of digestate can be used in many beneficial applications, such as animal bedding (solids), nutrient-rich fertilizer (liquids and solids), a foundation material for bio-based products (e.g. bioplastics), organic-rich compost (solids), and/or simply as soil amendment (solids), the latter of which may include the farm spreading the digestate on the field as fertilizer.

Although biogas production from organic substrates itself is a robust and efficient technology, the production costs are still higher than in the traditional power mix or the natural gas price. One cost driving factor is that biogas production through anaerobic digestion requires additional thermal energy in its operation, in particular for operating the anaerobic digester and post-processing of the resulting digestate.

It is known that energy conversion efficacy in biogas production can be significantly enhanced by using better energy recovery and thermal integration across different process steps. However, in particular waste heat recovery in biogas process is more complicated compared to other industrial processes. For one reason, the digestion process is usually operated at temperatures less than 70°C and the waste heat is low-grade. In addition, the heat recovery efficiency and the amount of waste heat that can be recovered strongly depend on multiple factors, including variations in ambient temperature and variations in compositions and moisture content of both the initial organic feedstock and the resulting digestate after anaerobic digestion.

There is thus not only a need for improving the energy efficiency of biogas production systems, but also for more flexibility in with respect to modes of its operation.

The problem solved by the present invention is therefore to provide a system and a method that both allow producing biogas in an energy efficient and economic manner and which enable a more flexible way of operation to react to changes in energy demands.

This problem is solved by the biogas production system according to Claim 1 and by the method for operating such a biogas production system according to Claim 11. Preferred embodiments are subject to the dependent claims.

In line with the present invention, a biogas production system is provided including an anaerobic digester configured for the production of a biogas and a digestate from an organic feedstock. The system further comprises a separator configured for separating at least part of the digestate from the digester into a liquid digestate fraction and a solid digestate fraction. The system also includes a thermal treatment unit configured for thermally treating at least part of the solid digestate fraction; a controller configured for controlling an operational parameter of the separator in response to at least one measured variable; and a sensor device configured for measuring - or determining - the at least one variable. The variable is indicative of the energy demand and/or the energy production of the system. The term "energy production" thereby specifically encompasses the production of waste heat, i.e. heat that is produced by an energy-consuming unit or process, as a by-product of doing work.

The digestate produced by the anaerobic digester is generally a combination of inert solids that were present in the organic feedstock, recalcitrant volatile solids that could not be degraded biologically, and bacterial biomass that grew as a result of feeding on the degradable portion of the organic feedstock. A typical digestate solids content is 2% to 10% total solids (TS) or dried solids (DS), depending on the substrate and the type of digester.

In case of the inventive system, the digestate is separated - usually mechanically - with the aid of a separator into a (more) liquid and a (more) solid digestate fraction. The terms "liquid" and "solid" digestate fraction are used to differentiate two fractions stemming from the same original digestate: a first fraction with a higher moisture content - the liquid digestate fraction - and a second fraction with a lower moisture content - the solid digestate fraction. As such, in comparison, the liquid digestate fraction has a higher moisture content than the solid digestate fraction, but solid parts can still be present in the liquid digestate fraction and the solid digestate fraction may also contain liquid components. The term "moisture content" relates to the amount of water contained in the digestate and is sometimes also referred to as "water content".

In accordance with the present invention, after the separation process, at least part of the solid digestate fraction is treated in a thermal treatment unit. The thermal treatment unit may be any kind of unit that allows a treatment at elevated temperatures. Preferably, the thermal treatment unit is selected from the group of pyrolysis unit, gasification unit, torrefaction unit, hydrothermal carbonization unit and hygienisation unit. Although it may also be used for other purposes, in the inventive system the thermal treatment unit is preferably configured for processing solid digestate material to produce liquid and gaseous fuel components, such as e.g. biochar and synthesis gas.

It is a key aspect of the present invention that a) at least one variable that is indicative of the energy demand and/or the energy production of the system is measured and that b) an operational parameter of the separator can be adjusted in response to the at least one measured variable, with the aim to increase the overall energy efficiency of the system. Specifically, the adjustment of the operational parameter shall impact the mode of operation and thereby the efficiency of the separator. The higher the efficiency of the separator, the better is the separation into the solid and liquid digestate fraction, i.e. the higher is the moisture content in the liquid digestate fraction and the lower is the moisture content in the solid digestate fraction. Examples of operational parameters that impact the efficiency of the separator are operational pressure, average or maximum speed, load, resistance, temperature etc.

The inventors realized that an adjustment of the efficiency of the separator has a direct impact on the energy demand of the system. That is because both the moisture content of the solid digestate fraction and the amount of liquid digestate fraction have a direct impact on the amount of energy required for storage and/or post-processing thereof. By adjusting one or more operational parameter(s) of the separator and thus the efficiency of the latter, the inventive system is provided with a high degree of flexibility to react to changing energy demands and energy production levels (including waste heat energy) of individual system components.

It was found that this newly gained flexibility allows to greatly optimize the overall energy consumption and energy distribution within the biogas production system. In particular, it enables efficient waste heat recovery and heat integration, that is the re-use of "waste heat", i.e. heat energy produced by units or processes within the system that would otherwise be disposed of or simply released into the atmosphere. Preferred ways of re-using waste heat include utilization of waste heat from the thermal treatment unit to reduce the moisture content of i) the solid digestate fraction prior to entering the thermal treatment unit and/or ii) the liquid digestate fraction to reduce the volume thereof.

In a preferred embodiment the at least one measured variable is selected from the group consisting of
i) moisture content in the organic feedstock,
ii) moisture content in the solid digestate fraction,
iii) ratio of the liquid digestate fraction to the solid digestate fraction,
iv) filling level of liquid digestate within a liquid digestate storage unit,
v) average temperature during the past 3 to 10 days,
vi) amount of heat energy produced in the thermal treatment unit,
vii) energy demands of energy consumers outside the biogas production system,
   and combinations thereof.

As will be explained in more detail below, each of the above-mentioned variables is indicative of an amount of energy required by any of the components of the system and/or an amount of energy produced by any of the system components that may potentially be extracted from the system, e.g. as waste heat.

Organic feedstock with a low moisture content generally has a high viscosity and thus generally requires more energy for mixing or transport thereof within the digester.

The moisture content in the solid digestate fraction impacts the amount of energy required for post-processing of the solid digestate fraction, in particular in post-processing involving drying and/or conversion of the digestate by heat treatment, such as hygienisation, pyrolysis, torrefaction, gasification or hydrothermal carbonization. The higher the moisture content, the more energy is required for such post-processing treatments.

The moisture content in a material can be determined by several methods. Typically, moisture content is determined via a thermogravimetric approach, i.e., by loss on drying, in which the sample is heated, e.g. in an oven, and the weight loss due to evaporation of moisture is recorded. Further methods are known to the skilled person as Pycnometer Method; Sand Bath Method; Alcohol Method; Calcium Carbide Method; and Radiation Method.

The ratio of liquid digestate fraction to solid digestate fraction refers to the volume ratio. The more efficient the separator operates, the larger is the volume of the liquid digestate fraction in relation to the volume of the solid digestate fraction. Liquid digestate is often stored in storage units before being transported to third-party recipients, e.g. for use as soil fertilizer. Storage and transport, however, are also energy-demanding factors. Therefore, the more liquid digestate is produced, the higher the costs associated with storage, transport and/or post-processing (drying, sanitizing etc.) thereof.

The filling level of liquid digestate within a liquid digestate storage unit is indicative of how much space is still available in the storage unit, i.e. until the maximum filling level is reached. Thus, if the filling level is high, it may be required to reduce the volume of the liquid digestate by drying - which is highly energy-consuming. Overall, one can say that a higher proportion of liquid digestate comes with an increased energy demand of the system.

The term "average temperature during the past 3 to 10 days" as used throughout this application refers to the ambient temperature at the location of the inventive system - or where the inventive method is used. Lower ambient temperatures generally come along with increased heating energy requirements, in particular heating energy required for heating the digester, the thermal treatment unit and/or other facilities of the biogas production system.

If energy is produced by any of the components of the inventive biogas production system - independent of its form (e.g. heat, compression, electricity...) - such energy may be extracted and stored and/or delivered to energy-demanding components within the biogas production system or to energy consumers outside the biogas production system. In the latter case, it is most common to extract energy and - if necessary - convert it into electricity that can then be fed into a local electricity grid.

In a preferred embodiment of the invention the separator includes a screw-press, and the controller is a pressure controller configured for controlling an operational pressure of the screw press in response to the at least one measured variable.

The operational pressure of the screw press has a direct impact on both the moisture content of the solid digestate fraction and the ratio of the liquid digestate fraction to the solid digestate fraction. As explained further above, both the moisture content of the solid digestate fraction and the amount of liquid digestate have a direct impact on the amount of energy required for storage and/or post-processing thereof.

In one embodiment, the operational pressure of the screw press is increased to decrease the moisture content in the solid digestate fraction and thus to reduce the energy required for post-processing of the solid digestate, in particular heat treatments involving pyrolysis, torrefaction, gasification or hydrothermal carbonization, which are used for converting the solid digestate fraction into valuable products, such as biochar or other high-energy fuel. This may be desirable if other components of the system have temporarily an increased energy demand, e.g. during cold weather periods that come with decreased ambient temperatures and therefore increased heating energy demands to keep operation temperatures within the digester and/or plant buildings.

In another embodiment, operational pressure of the screw press is adjusted to set the moisture content of the solid digestate fraction to a desired level, e.g. around 65-70%. This can be desirable to set optimum treatment conditions, e.g. treatment time and/or treatment temperature, for the thermal treatment of the solid digestate fraction, such that full conversion of the organic parts in the solid digestate, e.g. into biofuels, can be achieved. Stable heat treatment conditions bring the further benefit that the energy demand for the thermal treatment on the one hand, as well as the amount of resulting "waste heat" that can be used in internal thermal integration processes on the other hand can be estimated. The operational pressure may therefore be adjusted to optimize energy consumption of the thermal treatment unit with the aim to achieve essentially full conversion of the treated digestate and to improve utilisation of waste heat as well as overall energy efficiency of the system.

Instead of using a separator including a screw press as described above, it is alternatively preferred that the separator includes a sieving device comprising at least one vibrating sieve, and that the controller is a motor controller configured for controlling a vibration mode and/or exchange of the at least one sieve of the sieving device in response to the at least one measured variable.

If such a sieving device is used as a separator, the separation of into the two digestate fractions occurs by the liquid digestate fraction passing the sieve, whereas the solid digestate fraction is retained. The efficacy of the sieving device can be adjusted by adjusting the vibration mode, i.e. the vibration amplitude and/or vibration frequency, of the vibrating sieve and/or by adjusting the pore size of the sieve (said adjustment usually occurs via exchanging the sieve). More vigorous vibrating leads to more water passing the sieve, such that the moisture content of the solid digestate fraction decreases and the volume of the liquid digestate fraction increases. The opposite effect is achieved if the sieve is exchanged with a sieve having a smaller pore size. The smaller the pore size, i.e. the finer the mesh, of the sieve, the smaller the amount of liquid digestate that passes the sieve - and the bigger the amount of the solid digestate fraction that is retained by the sieve. Notably, a finer mesh generally not only retains more solid particles but also more water that sticks to the solid particles. Thus, with the aid of the motor controller, the amount liquid digestate passing the sieve as well as the amount and moisture content of the solid digestate fraction can be adjusted.

The reasons why it may be desirable to adjust the moisture content in the solid digestate and the amount of liquid digestate, respectively, have been explained above in connection with the embodiments that involve a separator including a screw press. These reasons apply also to embodiments that involve a separator including a sieving device.

In a preferred embodiment of the invention the separator and the thermal treatment unit are energetically connected to transfer energy from the thermal treatment unit to the separator. In this embodiment the term "energetically connected" refers to a connection that allows direct or indirect energy transfer from one component to another. In the specific case of energy transfer between the thermal treatment unit and the separator, the aim is to feed power to the separator. An example of a direct energy transfer means is a rigid or variable coupling in the sense of a mechanical coupling. An example of indirect energy transfer means is via the use of an energy converter, for instance a combined heat and power plant that produces electricity from waste heat produced in the thermal treatment unit and said electricity can then be used to drive the separator. In any case, by transferring energy from the thermal treatment unit to the separator, the latter requires less externally produced energy (i.e. electric energy or fuel produced outside of the biogas plant), which can reduce the overall biomethane production costs.

Preferably the thermal treatment unit is a pyrolysis unit or a gasification unit configured for thermally treating all or some of the solid digestate fraction at a temperature of at least 450°C, more preferably in a temperature range of 450°C to 1200°C.

Pyrolysis is a technique typically used to process solid biomass to produce carbonized products, liquids and gases by heating the biomass in a low or no oxygen environment. The absence or deficiency of oxygen prevents combustion. The relative yield of products from pyrolysis varies with temperature.

Pyrolysis of the digestate at at least 450°C enables effective conversion of the digestate into fully carbonized products, in particular solid products such as coke, carbon, charcoals and chars that can be used as such in industrial applications or as soil improver or as carbon sequestration means. Pyrolysis can also be used for the production of a synthesis gas, also called syngas or producer gas, which is a combination of CO, H₂ and CO₂ and which can serve as a fuel. Pyrolysis occurs more quickly at higher temperatures. In the high temperature range, e.g. above 700°C, conversion can be achieved within minutes or even seconds instead of hours.

High temperature pyrolysis is also known as gasification. Once initiated, both pyrolysis and gasification can be self supporting and produce net energy, not accounting for the energy value of the biomass consumed. In the literature, the distinction between pyrolysis and gasification is often blurry, although gasification is generally associated with higher temperatures than pyrolysis. Instead of calling it a pyrolysis unit, the thermal treatment unit may thus also be a gasification unit (or "gasifier") configured for thermally treating all or some of the solid digestate fraction at a temperature of at least 450°C. In case of a gasifier, it preferably enables a treatment at at least 600°C, more preferably at least 700°C, in particular in the range of 700-1200°C. Since higher temperatures favour the yield of liquid and gas fuel components, gasification primarily produces syngas. The newer process of fluidized-bed gasification can convert digestate into syngas in a time as short as one minute or even less. In addition, gasification, in particular fluidized bed gasification, has the benefit of being particularly effective in reducing the plastic content in the heat-treated digestate.

In an alternative preferred embodiment the thermal treatment unit is a hydrothermal carbonization unit configured for thermally treating all or some of the solid digestate fraction at a temperature of at least 175°C.

Hydrothermal carbonization has the benefit that it allows treatment of digestate with various moisture contents without pre-drying, which saves energy and related costs for drying. Similar to pyrolysis or gasification a thermal treatment involving a hydrothermal carbonization unit enables the recovery of the carbon contained in the organic feedstock via production of valuable products, such as hydrochar. Hydrochar can be further dried and pelletized for use in industrial applications.

A preferred treatment time in the hydrothermal carbonization unit is at least one hour, more preferably at least 2 hours, in particular 2-5 hours. While one hour heat treatment by hydrothermal carbonization has been found to be effective for converting at least part of the digestate into hydrochar, a treatment time of least 2 hours was found to allow effective conversion of the digestate into valuable products, such as high-quality fuel. The temperature for hydrothermal carbonization is preferably within the range of 175°C to 350°C, more preferably 175°C to 280°C.

In a preferred embodiment of the invention the system includes heat transfer means for transferring heat from the thermal treatment unit to a drying unit for at least partial drying of the solid digestate fraction, the liquid digestate fraction, or both. In other words, in this preferred embodiment thermal integration is carried out between the thermal treatment unit and a drying unit used for drying the solid and/or liquid digestate fraction. One preferred example of a heat transfer means is a heat exchanger. Heat from the thermal treatment unit may also be recovered using an economizer or waste heat boiler. With the aid of the drying unit, the moisture content and/or the overall amount of any of the two digestate fractions can be further reduced, independent of the operation mode of the separator, e.g. independent of the pressure used in the screw press. As mentioned, this may be desirable to reduce or stabilize the energy demand of a subsequent thermal treatment that is carried out for e.g. conversion and/or hygienisation of the digestate. Reducing the amount of liquid digestate can is generally beneficial since it is often stored or transported prior to using it e.g. as fertilizer. Thus, a reduced amount of liquid digestate means a reduction of costs associated with such storage or transport.

In a more specific preferred embodiment, the thermal treatment unit is a pyrolysis or gasification unit that is configured for converting at least part of the solid digestate fraction into a syngas of elevated temperature, wherein heat from the syngas is recovered and transferred to the drying unit. As mentioned further above, common means for heat recovery include heat exchanger, economizers and waste heat boilers. As a more specific example, a gas/liquid heat exchanger can be used for recovering heat from the syngas. The heat recovered as hot water can e.g. be used in a low temperature direct belt dryer in the drying unit. Another option to recover heat from the syngas for drying purposes is to use a gas/gas heat exchanger, wherein syngas heat is transferred to air used in the belt dryer.

In a preferred embodiment of the invention the separator and the anaerobic digester are connected via a transfer pipe to allow transfer of some or all of the liquid digestate fraction back into the digester. The presence of the transfer pipe enables transferring at least a part of the liquid digestate fraction back into the digester to decrease the viscosity of the digestate within the digester. This can be beneficial for example when the viscosity of the digestate within the digester is too high, which leads to an increased the wear of drives and gearboxes and an increased energy consumption of the mixing process in the digester.

Preferably the system includes a pre-treatment unit for decreasing the viscosity of the organic feedstock. The pre-treatment unit preferably includes means for decreasing the particle size of the organic feedstock, preferably by shredding, chopping, grinding, sieving and combinations thereof. As mentioned before, the reduction of the particle size of the organic feedstock will also lead to a decreased viscosity of the digestate, which results in a reduced energy consumption and a reduced wear on any agitator or mixing device used within the digester. Further the use of the pre-treatment unit enables to reduce the viscosity of the digestate without or with less addition of dilution liquids. This reduces the likelihood of sedimentation occurring within the digester, which allows for a better separation of solids and liquid digestate in the separator. In particular in case of the separator including a screw press, a reduced viscosity of the digestate reduces the screw press' energy consumption and wear.

Another aspect of the invention is the provision of a method for operating a biogas production system including the following steps: In a first step a) an organic feedstock is fed into an anaerobic digester for the production of biogas and a digestate. In a second step b) at least part of the digestate from the digester is separated into a liquid digestate fraction and a solid digestate fraction with the aid of a separator. In a third step c) at least part of the solid digestate fraction is thermally treated in a thermal treatment unit. In a fourth step d) at least one variable is measured with the aid of a sensor device. Said variable is indicative of the energy demand and/or the energy production (in particular the waste energy production) of the system. In a fifth step e) an operational parameter of the separator is adjusted with the aid of a controller in response to the at least one measured variable.

Analog to the benefits mentioned above in connection with the inventive system, an adjustment of the operational parameter of the separator in response to the one or more measured variable(s) allows for thermal integration between the different steps and for improving the overall energy efficiency of the inventive method.

In the following, preferred embodiments of the inventive method will be described. Many of the features that are preferred for the inventive method have already been discussed above in connection with the inventive system. For the effects and benefits associated with preferred features that are shared by both the system and the method of the present invention it is therefore referred to the pertaining paragraphs above.

Analogue to the preferred embodiments of the inventive system, the at least one measured variable is preferably selected from the group consisting of
i) moisture content in the organic feedstock,
ii) moisture content in the solid digestate fraction,
iii) ratio of the liquid digestate fraction to the solid digestate fraction,
iv) filling level of liquid digestate within a liquid digestate storage unit,
v) average temperature during the past 3 to 10 days,
vi) amount of heat energy produced in the thermal treatment unit,
vii) energy demands of energy consumers outside the biogas production system,
   and combinations thereof.

In a preferred embodiment the separation of the digestate in step b) is accomplished with the aid of a screw press and wherein in step e) a pressure controller is used for adjusting an operational pressure of the screw press in response to the at least one measured variable. Further details concerning the use of a separator that includes a screw press have been given further above in connection with the pertaining preferred embodiments of the inventive system.

In case of using a screw press as separator it is further preferred that step d) involves measuring the water content in the solid digestate fraction and step e) involves increasing the operational pressure of the screw press if the water content of the solid digestate fraction is above 75%. Generally, the moisture content of the solid digestate fraction is kept below 75%, more preferably within the range 65-70%. Thereby, the volume and therefore the space requirement of the solid digestate fraction can be reduced, and thereby the energy required for storage, transport or subsequent treatment, in particular the subsequent thermal treatment of (at least part of) the solid digestate, can be controlled.

Instead of using a screw press as a separator it is alternatively preferred that the separation of the digestate in step b) is accomplished with the aid of a sieving device comprising at least one vibrating sieve, and wherein in step e) a motor controller is used for adjusting a vibration mode and/or exchange of the at least one sieve of the sieving device in response to the at least one measured variable. For further details concerning the use of a sieving device it is referred to the paragraphs discussing the preferred embodiment of the inventive system that includes a sieving device.

Independent of the type of the separator it is preferred that in step c) of the inventive method, some or all of the solid digestate fraction is thermally treated in a pyrolysis unit or a gasifier unit at a temperature of at least 450°C for at least 15 minutes, preferably 15 to 180 minutes, more preferably 20 to 140 minutes. Pyrolysis of the digestate at 450°C or more for at least 15 minutes was found to enable effective conversion of the digestate into fully carbonized products, such as coke, carbon, charcoals and chars that can be used as such in industrial applications or as soil improver or as carbon sequestration means. Alternatively, all or some of the solid digestate fraction can be treated by gasification at a temperature of at least 450°C, preferably at least 600°C, more preferably at least 700°C, in particular in the range of 700-1200°C, to (primarily) produce a syngas. For syngas production the minimum treatment time of the digestate highly depends on the type of gasification. Traditional solid-bed or fixed-bed gasification often requires a longer treatment time than a few minutes, but highly depends on the type and moisture content of the digestate. Pre-drying of the digestate can be employed to reduce the minimum treatment time. The newer process of fluidized-bed gasification can convert digestate into syngas in a time as short as one minute or even less. In addition, it was found that gasification, in particular fluidized bed gasification, has the benefit of being particularly effective in reducing the plastic content in the heat-treated digestate.

As alternative to a treatment by pyrolysis or gasification, it is preferred that in step c), some or all of the solid digestate fraction is thermally treated in a hydrothermal carbonization unit at a temperature of at least 175°C for at least 1 hour, preferably at least 2 hours, to produce carbonized products - in case of hydrothermal carbonization those are usually bio-coal and a residual liquid. The temperature for hydrothermal carbonization is preferably within the range of 175°C to 350°C, more preferably 175°C to 280°C. Hydrothermal carbonization has the benefit that it allows treatment of digestate with various moisture contents without pre-drying, which saves energy and costs for drying before processing. The produced bio-coal can serve as high quality fuel.

If the inventive method involves hydrothermal carbonization it is preferred that the bio-coal and the residual liquid are separated, preferably with the aid of a filter press, and at least part of the residual liquid is re-introduced into the anaerobic digester. Additionally or alternatively, it is preferred that at least part of the liquid digestate fraction is re-introduced into the anaerobic digester. As mentioned in connection with the related preferred embodiment of the inventive system, a re-introduction of at least some residual liquid and/or of at least some of the liquid digestate into the anaerobic digester enables to decrease the viscosity of the digestate within the digester. This can be beneficial for example when the viscosity of the digestate within the digester is too high, which leads to an increased the wear of drives and gearboxes and an increased energy consumption of the mixing process in the digester.

In a preferred embodiment of the inventive method, heat energy from the thermal treatment unit is extracted, e.g. with the aid of a heat exchanger, and used for drying any of the separated digestate fractions. As mentioned, additional drying of the digestate fractions reduces the costs involved with subsequent treatments, storage and/or transport thereof. By using heat energy from the thermal treatment unit for the drying, the overall energy efficiency of the inventive method is improved. The heat energy from the thermal treatment unit may also be used for heating up the organic feedstock - either prior to entering the digester or within the digester.

If the thermal treatment involves pyrolysis or gasification, it is preferred that the thermal treatment is used for converting at least part of the solid digestate fraction into a syngas of elevated temperature. Heat - or specifically waste heat - of said syngas is subsequently preferably recovered, e.g. with the aid of a heat exchanger, economizer or waste heat boiler, and used for other processes within the system, such as for heating the anaerobic digester or for drying the solid and/or liquid digestate fraction. Production of electricity from the waste heat of the syngas, e.g. with the aid of a heat-and-power-unit is also an option. Said electricity is then preferably used for driving other energy-consuming units within the system.

It is further preferred that the particle size of the organic feedstock is decreased, preferably by shredding, grinding or sieving prior to feeding the organic feedstock into the anaerobic digester. This results in a reduced energy consumption and a reduced wear on any agitator or mixing device used within the anaerobic digester and the screw press.

### Description of figures

The present invention will now be described, by way of two examples, with reference to the accompanying drawings in which:
- Fig. 1: shows a biogas production system including a screwpress in accordance with a first embodiment of the present invention.
- Fig. 2: shows a biogas production system including a sieving device in accordance with a second embodiment of the present invention.

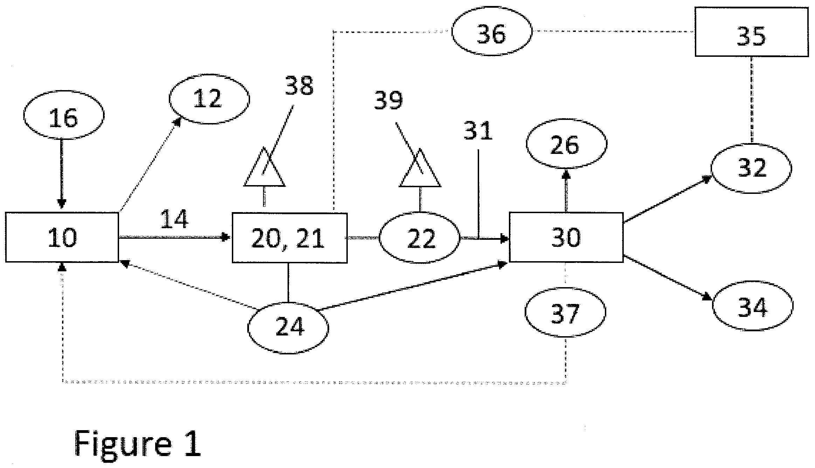

Fig. 1 shows the components of a first preferred embodiment of a biogas production system in accordance with the present invention. Said system includes an anaerobic digester 10 configured to produce methane-containing biogas 12 and a digestate 14 through anaerobic digestion of organic feedstock 16 through microorganisms in the absence of oxygen. The organic feedstock 16 can be a variety of usually carbon-rich materials, such as urban wood waste, paper waste, cow manure, food waste, agricultural waste, etc. Although not shown in the schematic figure, the anaerobic digester 10 comprises an inlet for receiving the organic biomass 16, an outlet for discharging digestate 14, and a temperature controller to control the temperature within the digester 10. The digester 10 may be a plug-flow digester and may include agitators to aid mixing of the digestate within.

The system further includes transfer or transportation means (not shown) for supplying at least part of the digestate 14 that is discharged from the digester 10 to a separator 20. The separator 20 includes a screw press 21 that separates the digestate 14 from the digester 10 into a solid digestate fraction 22 and a liquid digestate fraction 24.

The liquid digestate fraction 24 is collected and some of it is used for humidification of the digester feedstock 16, meaning that some of the liquid digestate fraction 24 is returned to the digester 10 for increasing the moisture content (sometimes also referred to as "water content") of the organic feedstock 16 within the digester 10. This adjustment of the moisture content and thus the viscosity of the digestate 14 within the digester 10 avoids excessive energy consumption for mixing or agitating the digestate 14 and also reduces the wear on the mixing/agitation means.

At least part of the solid digestate fraction 22 is supplied to a thermal treatment unit 30, e.g. through transfer pipes 31 connecting the separator 20 and the thermal treatment unit 30. At least part of the liquid digestate fraction 24 may also be heat-treated in the thermal treatment unit 30 for the purpose of being reduced in moisture content and/or being hygienized, meaning that any pathogens other harmful organisms are effectively killed or at least deactivated. The treated liquid digestate 26 is in this case released from the thermal treatment unit 30 and can be used e.g. as fertilizer.

The thermal treatment unit 30 may be any kind of unit that allows a treatment of the solid digestate 22 (and optionally the liquid digestate) at elevated temperatures. In most cases the thermal treatment unit 30 is one of a pyrolysis reactor, a gasification reactor, a torrefaction reactor or a hydrothermal carbonization reactor. In the shown embodiment, it is a gasification reactor (also called gasifier). Within the gasifier 30, the solid digestate fraction 22 is heated to at least 700° for a few minutes, during which the solid digestate fraction 22 is converted into a synthesis gas (syngas 32) and biochar 34. The syngas 32 can be burned directly in gas engines, used to produce methanol and hydrogen, or converted via the Fischer-Tropsch process into synthetic fuel. A heat-to-power-unit 35 is provided to recover waste heat from the syngas 32 and convert it into electricity 36 that is used for driving the separator 20 (any transfer of waste heat or electricity is indicated in the figure by a dashed line). The gasifier 30 is preferably a fluidized bed reactor, specifically one that utilizes a pressurized reactor containing a fluid bed of sand, known as a pressurized bubbling fluidized bed (PBFB). To further improve the energy balance of the system, waste heat 37 from the gasifier 30 is recovered and at least partly transferred to the digester 10. Heat recovery can occur in any manner known to the person skilled in the art, e.g. with the aid of a heat exchanger, economizer, waste heat boiler etc.

The system further includes a pressure controller 38 configured for controlling an operational pressure of the screw press 21 in response to at least one measured variable. In the shown embodiment, the operational pressure of the screw press 21 is adjusted in response to the moisture content of the solid digestate fraction 22. This moisture content is measured with the aid of a sensor device 39. Knowing the moisture content of the solid digestate fraction 22 allows decreasing or increasing the operational pressure of the screw press 21 to a desired value. Said desired value can be set to optimize energy consumption of certain system components and/or to optimize the overall energy efficiency of the system.

In the embodiment shown in Fig. 1, the operational pressure is continuously adjusted to keep the moisture content of the solid digestate fraction 22 at around 65-70%. This allows setting an optimum treatment time and/or treatment temperature in the thermal treatment unit 30 for converting the solid digestate 22 into syngas 32 and biochar 34. This itself significantly improves the overall energy efficiency of the system. In addition to the above-described recovery of waste heat from the syngas 32 to produce electricity 36, part of said recovered waste heat may also be used for heating the fermenter 10 (not shown) .

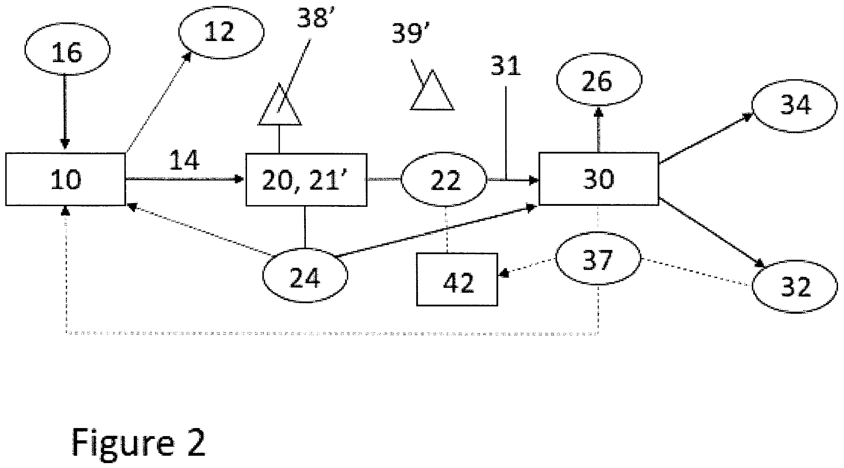

Fig. 2 shows a second preferred embodiment of a biogas production system in accordance with the present invention. The systems shown in Fig. 1 and 2 are similar and differ only in a few aspects. The following description of Fig. 2 therefore limited to the differentiating aspects, whereas for all other components that both embodiments have in common (labelled with the same reference numbers) it is referred to the above description in connection with Fig. 1.

The embodiment shown in Fig. 2 differs from the one in Fig. 1 in the type of separator used for separating the digestate into a more solid and a more liquid digestate fraction, respectively. They also differ in the measured variable based on which an operational parameter of the separator is adjusted. A third difference is the involvement of a drying unit and utilization of waste energy.

In the embodiment of Fig. 2, the separator 20 includes a sieving device 21' with at least one vibrating sieve. The system further includes a motor controller 38' that is used for adjusting a vibration mode of the sieving device 21' and/or exchange of the at least one sieve of the sieving device in response to the at least one measured variable. In this embodiment, the measured variable is the average ambient temperature during the past 3 to 10 days, specifically the last 5 days. This average temperature during the past 5 days is measured with the aid of a temperature sensor device 39'.

If the measured average ambient temperature is higher than a certain value, e.g. a long-time average reference value, this indicates that less energy is required for heating the digester 10 and/or facility buildings within the system. There is thus a decreased energy demand from some components in the system. In reaction thereof, the system can be adjusted to redirect more or less energy, in particular available waste heat 37 from the thermal treatment unit 30 or other components, to other areas or processes in the system. In the shown embodiment, some of the waste heat 37 recovered from the thermal treatment unit 30 and from the syngas 32 can be transferred to a drying unit 42 that is used to dry the solid digestate fraction 22 prior to entering the thermal treatment unit 30. The pre-drying reduces the moisture content of the solid digestate fraction. If more energy / waste heat 37 is available for drying the solid digestate fraction 22, the efficiency of the separator 20 can be lowered. An increased amount of solid digestate fraction 22 with a higher moisture content and a reduced amount of liquid digestate fraction 24 will be the result. If the separator includes a sieving device 21' as in the embodiment shown in Fig. 2, a lower efficiency can be achieved by decreasing the vibration amplitude and/or vibration frequency of the vibrating sieve and/or by exchanging the sieve with another sieve having a smaller the pore size. This adjustment of the vibrating mode and/or the sieve exchange is controlled by means of the motor controller 38'. The benefit of such an adjustment is the reduced amount of liquid digestate, if e.g. if storage space in a liquid digestate storage tank (not shown) is limited.

Some or all of the recovered waste heat 37 may also be used for heating the fermenter 10.

## Claims

1. Biogas production system including
an anaerobic digester (10) configured for the production of a biogas (12) and a digestate (14) from an organic feedstock (16);
a separator (20, 21, 21') configured for separating at least part of the digestate (14) from the digester (10) into a solid digestate fraction (22) and a liquid digestate fraction (24);
a thermal treatment unit (30) configured for thermally treating at least part of the solid digestate fraction (22) ;
a controller (38, 38') configured for controlling an operational parameter of the separator (20) in response to at least one measured variable that is indicative of the energy demand and/or the energy production of the system; and
a sensor device (39, 39') configured for measuring the at least one variable.

2. Biogas production system according to Claim 1, wherein the at least one measured variable is selected from the group consisting of
i) moisture content in the organic feedstock (16),
ii) moisture content in the solid digestate fraction (22),
iii) ratio of the liquid digestate fraction (24) to the solid digestate fraction (22),
iv) filling level of liquid digestate within a liquid digestate storage unit,
v) average temperature during the past 3 to 10 days,
vi) amount of heat energy produced in the thermal treatment unit (30),
vii) energy demands of energy consumers outside the biogas production system,
and combinations thereof.

3. Biogas production system according to Claim 1 or 2, wherein the separator (20) includes a screw-press (21), and the controller is a pressure controller (38) configured for controlling an operational pressure of the screw press (21) in response to the at least one measured variable.

4. Biogas production system according to Claim 1 or 2, wherein the separator (20) includes a sieving device (21') comprising at least one vibrating sieve, and the controller is a motor controller (38') configured for controlling a vibration mode and/or exchange of the at least one sieve of the sieving device (21') in response to the at least one measured variable.

5. Biogas production system according to any of Claims 1 to 4, wherein the separator (20, 21, 21') and the thermal treatment unit (30) are energetically connected to transfer energy from the thermal treatment unit (30) to the separator (20, 21, 21').

6. Biogas production system according to any of Claims 1 to 5, wherein the thermal treatment unit (30) is a pyrolysis unit or a gasification unit configured for thermally treating all or some of the solid digestate fraction (22) at a temperature of at least 450°C.

7. Biogas production system according to any of Claims 1 to 6, wherein the thermal treatment unit (30) is a hydrothermal carbonization unit configured for thermally treating all or some of the solid digestate fraction (22) at a temperature of at least 175°C.

8. Biogas production system according to any of Claims 1 to 7, wherein the system includes heat transfer means for transferring heat from the thermal treatment unit (30) to a drying unit (42) for at least partial drying any of the digestate fractions (22, 24).

9. Biogas production system according to any of Claims 1 to 8, wherein the separator (20, 21, 21') and the anaerobic digester (10) are connected via a transfer pipe to allow transfer of at least part of the liquid digestate fraction (24) back into the digester.

10. Biogas production system according to any of Claims 1 to 9 wherein the system includes a pre-treatment unit for decreasing the viscosity of the organic feedstock; the pre-treatment unit including means for decreasing the particle size of the organic feedstock, preferably by shredding, grinding, chopping, sieving and combinations thereof.

11. Method for operating a biogas production system as claimed in any of Claims 1 to 10, including the steps of
a) Feeding an organic feedstock (16) into an anaerobic digester (10) for the production of biogas (12) and a digestate (14);
b) With the aid of a separator (20, 21, 21'), separating at least part of the digestate (14) from the digester (10) into a solid digestate fraction (22) and a liquid digestate fraction (24);
c) Thermally treating at least part of the solid digestate fraction (22) in a thermal treatment unit (30);
d) With the aid of a sensor device (39, 39'), measuring at least one variable that is indicative of the energy demand and/or the energy production of the system; and
e) Adjusting an operational parameter of the separator (20, 21, 21') with the aid of a controller (38, 38') in response to the at least one measured variable.

12. Method according to Claim 11, wherein the at least one variable measured in step d) is selected from the group consisting of
i) moisture content in the organic feedstock (16),
ii) moisture content in the solid digestate fraction (22),
iii) ratio of the liquid digestate fraction (24) to the solid digestate fraction (22),
iv) filling level of liquid digestate within a liquid digestate storage unit,
v) average temperature during the past 3 to 10 days,
vi) amount of heat energy produced in the thermal treatment unit (30),
vii) energy demands of energy consumers outside the biogas production system,
and combinations thereof.

13. Method according to Claim 11 or 12, wherein the separation of the digestate (14) in step b) is accomplished with the aid of a screw press (21) and wherein in step e) a pressure controller (38) is used for adjusting an operational pressure of the screw press (21) in response to the at least one measured variable.

14. Method according to Claim 13, wherein step d) involves measuring the moisture content in the solid digestate fraction (22) and step e) involves increasing the operational pressure of the screw press (21) if the moisture content of the solid digestate fraction (22) is above 75%.

15. Method according to Claim 11 or 12, wherein the separation of the digestate (14) in step b) is accomplished with the aid of a sieving device (21') comprising at least one vibrating sieve, and wherein in step e) a motor controller (38') is used for adjusting a vibration mode and/or exchange of the at least one sieve of the sieving device (21') in response to the at least one measured variable.

16. Method according to any of Claims 11 to 15, wherein heat energy from the thermal treatment unit (30) is used for drying any of the separated digestate fractions (22, 24) and/or for heating the organic feedstock (16).
